Europäisches Patentamt

European Patent Office

Office européen des brevets

(11) Veröffentlichungsnummer: **0 002 509**
**A1**

# (12) EUROPÄISCHE PATENTANMELDUNG

(21) Anmeldenummer: **78101619.1**

(22) Anmeldetag: **08.12.78**

(51) Int. Cl.²: **A 61 F 13/02**
**A 61 L 15/06**

(30) Priorität: **08.12.77 SE 7713918**

(43) Veröffentlichungstag der Anmeldung:
**27.06.79 Patentblatt 79/13**

(84) Benannte Vertragsstaaten:
**BE CH DE FR GB NL**

(71) Anmelder: **Mo och Domsjö Aktiebolag**
**Fack**
**S-89101 Örnsköldsvik(SE)**

(72) Erfinder: **Hessner, Hans**
**Ymervägen 56**
**S-182 63Djursholm(SE)**

(74) Vertreter: **Schmied-Kowarzik, Volker, Dr et al,**
**Patentanwälte P. Wirth V. Schmied-Kowarzik, G.**
**Dannenberg P. Weinhold, D. Gudel Siegfriedstrasse 8**
**D-8000 München 40(DE)**

(54) Verband zur Aufbringung auf Wunden.

(57) Verband zur Aufbringung auf Wunden (3), insbesondere mit starker Flüssigkeitssekretion, umfassend in Kombination:

a) zwei Klebestücke (1, 2) mit halbkreisförmigen Aussparungen zum Aufkleben auf die Haut oder gegebenenfalls teilweise aufeinander, so daß die halbkreisförmigen Aussparungen die Wunde einschließen,

b) eine vorzugsweise durchsichtige, perforierte Folie oder ein Netz (4), das auf den Klebestücken (1, 2) und gegebenenfalls auch außerhalb der Klebestücke (1, 2) angeordnet ist und an diesen und/oder der Haut außerhalb der Klebestücke (1, 2) befestigt wird,

c) einen austauschbaren Absorptionskörper (5) von bezüglich der Wunder konvexer Form, bestehend aus einem Absorptionskern (8), der mindestens teilweise und in dem Gebiet um die Wunde aus einem Material (10) mit einer Wasserabsorption von mindestens 50 mm pro 10 Minuten, gemessen gemäß Klemm (SCAN-P 13:64) besteht und einer flüssigkeitsdurchlässigen, den Absorptionskern (8) einschließenden Schicht (9), wobei der Absorptionskörper (5) auf der an die Wunde anschließenden Folie oder dem Netz (4) angeordnet und mittels Klebestücken (6, 7) in dieser Stellung befestigt ist.

Fig. c

EP 0 002 509 A1

Die vorliegende Erfindung bezieht sich auf einen Verband zur Aufbringung auf Wunden, vorzugsweise auf Wunden mit starker Flüssigkeitssektretion.

Zu diesem Zweck wurden früher viele Arten von Verbänden verwendet, und neuerdings hat die Verwendung hoch absorbierender, pulver- bzw. puderartiger Materialien, wie Dextran, eingesetzt. Das von der Firma AB Pharmacia als DEBRISON $^R$ verkaufte Dextranprodukt ist ein Beispiel eines solchen puderartigen Materials von hoher Absorptionskraft. Der Puder wird direkt auf die Wunde aufgebracht und in regelmäßigen Abständen gewechselt. Solange die Wunde feucht ist, kann der Puder ohne Beschwerden gewechselt werden, wenn jedoch zwischen zwei Wechsel eine zu lange Zeit vergeht, d.h. wenn die Puderkörner mit Wundenflüssigkeit gesättigt sind, dann bildet sich eine Kruste, die entfernt werden muß. Um dies zu vermeiden, muß der Puder oft gewechselt werden, was wiederum zu einem hohen Puderverbrauch führt; da dieser sehr kostspielig ist, bedeutet dies sehr hohe Gesamtkosten für das Heilen der Wunde. Um die Heilkosten einer Wunde mit starker Flüssigkeitssekretion zu verringern und mögliche Probleme beim Puderwechsel zu vermeiden, wurde der vorliegende Verband entwickelt. Durch ihn wurde es möglich, den Verband auf einer Wunde ohne Beschädigung der Wundenoberfläche zu ersetzen. Der erfindungsgemäße Verband umfaßt in Kombination

a) zwei mit halbkreisförmigen Aussparungen ("crescent-shaped recesses") versehene Klebestücke bzw. -bänder, die zur Aufbringung bzw. zum Ankleben auf die Haut und gegebenenfalls aufeinander geeignet sind, so daß die halbkreisförmigen Aussparungen die Wunde einschließen,

b) eine vorzugsweise durchsichtige, perforierte Folie oder ein Netz, das zum Auflegen auf und gegebenenfalls außerhalb der Klebestücke geeignet ist und an den Klebestücken und/oder an der Haut außerhalb der Klebestücke befestigt werden kann.

c) einen austauschbaren Absorptionskörper von konvexer Form in Bezug zur Wunde, bestehend aus einem Absorptionskern, der mindestens teilweise und im Gebiet um die Wunde aus einem Material mit einer Wasserabsorption von mindestens 500 mm pro 10 Minuten, gemessen nach Klemm (SCAN-P 13:64), und einer den Absorptionskern einschließenden, flüssig-keitsdurchlässigen Schicht besteht, wobei der Absorptionskörper auf die an die Wunde anschließende Kunststofffolie oder das Kunststoffnetz gelegt und in dieser Stellung mittels eines Klebestückes bzw. Pflasters verankert werden kann.

Der erfindungsgemäße Verband wird in solcher Weise verwen-det, daß die Klebestücke /entsprechend a) und das Material
                              bzw. Pflaster
entsprechend b) längere Zeit auf dem Träger stationär gehalten werden, während der Absorptionskörper gemäß c) nach Bedarf gewechselt wird.


Der Verband wird in den beiliegenden Zeichnungen a bis c genauer beschrieben.

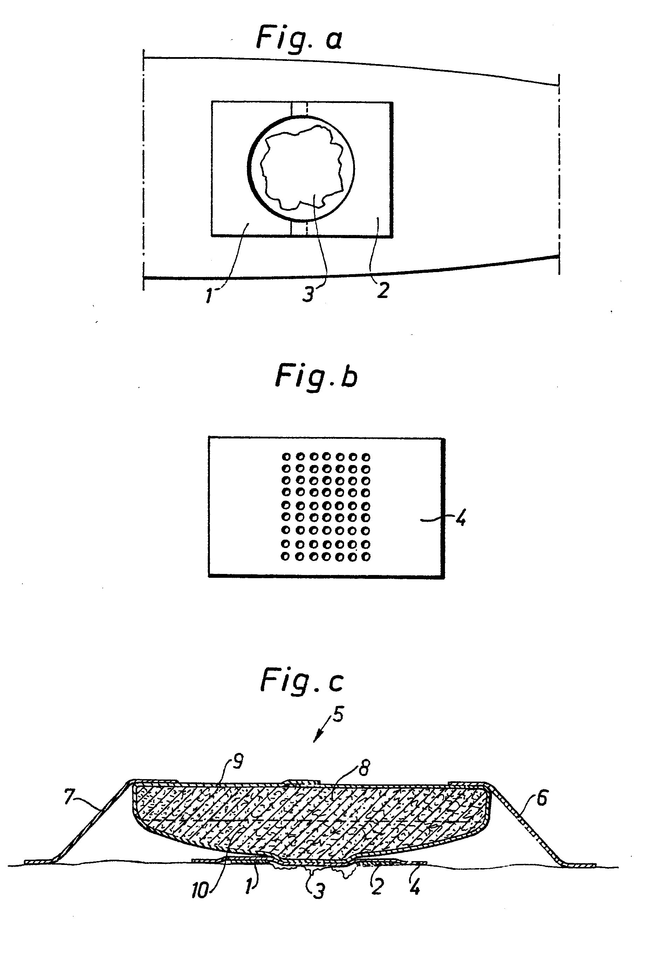

Fig. a) zeigt zwei Klebestücke 1 und 2, die eine Wunde 3, z.B. auf einem Arm, einschließen. Die Klebestücke haben halbkreisförmige Aussparungen und werden so auf die Haut geklebt, daß die Wunde vollständig eingeschlossen ist. Die Klebestücke können so geformt sein, daß sie entweder Kante an Kante auf die Haut geklebt werden oder, wie in der Zeichnung dargestellt, daß die Enden der beiden Klebestücke gegenseitig überlappen. Die Enden können auch in stärkerem Maß als in der Zeichnung dargestellt überlappen, was jeweils von der Form der Wunde abhängt. Wichtig ist, daß die Wunde vollständig eingeschlossen ist, und daß die Klebestücke weiterhin möglichst nahe an die Wundkante ohne Gefahr einer Entzündung der Wunde angelegt werden. So eliminiert man die Gefahr einer Infektion der Wunde durch Bakterien, die in der nächsten Umgebung der Wunde gebildet werden. Diese Probleme bestanden früher bei Verbänden, die eine wärme und feuchte Atmosphäre in Wundennähe ergeben. Die Klebestücke sollen so dünn sein, daß sie der Form des Körperteiles leicht folgen können. Die Klebestücke können einfach- oder doppel-haftend sein. Die Haftsubstanz sollte vor Aufbringung der Klebestücke auf den Träger durch ein Trennmaterial abgedeckt werden. Dieses Trennmaterial kann z.B. ein siliconbehandeltes Papier sein, das bei Aufbringung der Klebestücke um die Wunde leicht entfernt wird. Die Klebestücke können aus einem für solche Zwecke allgemein verwendeten Material hergestellt werden und z.B. aus mit Binder überzogenen Kunststoffolien oder gewebtem Material bestehen. Erfindungsgemäß wird jedoch vorzugsweise ein Stück aus einem dampfdurchlässigen Material verwendet, z.B. ein nicht-gewebtes, faserartiges Material, das auf einer oder beiden Seiten mit einem Bindemittel überzogen und gegebenenfalls mit einem Schutzpapier versehen ist. Ein derartiges, geeignetes Band ist als MICROPORE [R] von der Firmel Minnesota Mining Co. im Handel.

Auf die Klebestücke 1 und 2 wird eine dünne, vorzugsweise durchsichtige Folie oder ein Netz 4 gelegt. Die Folie oder das Netz kann aus einem Kunststoff oder gewebten Material, Tiergewebe (Collagen) oder einem anderen, für diesen Zweck geeigneten Material bestehen; im Fall einer Folie sollte sie, wie in Fig. b) ersichtlich, mit einer großen Anzahl nahe beieinander liegender Perforationen versehen sein. Diese können z.I rund oder oval sein und werden zentral auf der Folie angebracht, so daß sie die Wunde einschließen oder noch besser etwas größer als die Wundenoberfläche sind. Anstelle einer Folie mit Perforationen kann, wie oben erwähnt, ein Netz verwendet werden. Wenn die Klebestücke gemäß Fig. a) doppelhaftend sind, dann erzielt man automatisch eine Haftung zwischen diesen und der Folie, wenn diese auf die Wunde aufgebracht wird, nachdem das Trennmaterial auf den Klebestücken entfernt wurde. Sind die Klebestücke nicht doppelhaftend, dann können die Kanten der Folie mit einem Bindemittel versehen sein, das die Folie an den Klebestücken und/oder den umgebenden Hautteilen haften läßt. Weiter kann man im Voraus die Folie oder das Netz an einem der Klebestücke befestigen, so daß man bei Aufbringung des Verbandes zuerst ein Klebestück auf eine Seite der Wunde legt und dann das zweite Klebestück mit haftender Folie oder Netz in Verbindung mit dem ersten Klebestück bringt, so daß die Wunde von der Folie oder dem Netz vollständig bedeckt wird. Wie oben erwähnt, sollen Folie oder Netz vorzugsweise durchsichtig und so weitgehend perforiert sein, daß das Aussehen der Wunde jederzeit untersucht werden kann. Die Folie oder das Netz 4 soll zweckmäßig so aufzubringen sein, daß der perforierte Teil gegen die Wunde eine konvexe Kurve beschreibt.

Fig. a) und b) zeigen die Klebestücke 1 und 2 bzw. die Folie 4 von oben, während Fig. c) die Klebestücke, die Folie und den Absorptionskörper 5 nach Aufbringung auf die Wunde von der Seite zeigt. Der Absorptionskörper 5 hat, wie aus der Zeichnung ersichtlich, eine konvexe Form und die gekurvte Seite wird auf die Folie 4 und gegen die Wunde 3 gelegt. Der Absorptionskörper wird durch zwei Pflaster 6 und 7 an Ort und Stelle gehalten, die teilweise auf dem Absorptionskörper und teilweise auf der Haut des Patienten befestigt werden. In der Zeichnung werden zwei Pflaster 6 und 7 gezeigt, die an jedes Ende des Absorptionskörpers festgemacht sind. Diese beiden Pflaster können jedoch auch durch 1 Pflaster ersetzt werden, das hinter einem Ende des Absorptionskörpers eine Lasche bildet und sich entlang des gesamten Absorptionskörpers erstreckt und an diesen sowie an einer Lasche außerhalb des anderen Endes des Absorptionskörpers befestigt wird. Die Seite des oder der Pflaster, die auf beiden Seiten der Wunde gegen die Haut gerichtet sind, ist mit einem Bindemittel und einem schützenden Trennmaterial versehen, der unmittelbar vor der Aufbringung des Absorptionskörpers auf die Wunde entfernt wird. Die konvexe Form des Absorptionskörpers wirkt der gewöhnlich an der Wunde auftretenden Eiterung ("gathering") entgegen und ermöglicht auch einen guten Kontakt zwischen der Wundenoberfläche und dem Absorptionskörper, so daß die Absorption der Wundenflüssigkeit erleichtert wird. Die konvexe Form des Absorptionskörpers 5 kann variiert werden, in Fig. c) wird jedoch eine geeignete Konvexform in Längsrichtung des Absorptionskörpers gezeigt. Die Konvexform in Querrichtung des Absorptionskörpers kann in derselben Weise wie in der Längsrichtung variiert werden. vom Herstellungsstandpunkt wird jedoch eine geringere Konvexform in Querrichtung des Absorptionskörpers derjenigen in Längsrichtung bevorzugt.

Der Absorptionskörper 5 besteht aus einem Absorptionskern 8 mit einer einhüllenden, flüssigkeitsdurchlässigen Schicht 9. Eine Forderung an die Schicht 9 besteht darin, daß sie die Wundenflüssigkeit ohne Behinderung durchläßg und gleichzeitig stark genug ist, um den Absorptionskörper zusammenzuhalten.

Weiter darf das Material keine losen Fasern oder faserartigen Flaum abgeben. Geeignet sind z.B. nicht-gewebte Materialien von unterschiedlicher Art. Der Absorptionskern 8 kann aus verschiedenen absorbierenden Materialien aufgebaut sein. Bezüglich der der Wunde 3 am nächsten liegenden Schicht/des (10) Absorptionsmaterials ist zu fordern, daß das Material eine als Saughöhe bezeichnete Wasserabsorptionskraft von mindestens 50 mm pro 10 Minuten, gemessen gemäß Klemm, hat. Dieses Meßverfahren ist in SCAN-P 13/64 (Papper och Trä 46; (1964) 10, Seite 603-605) beschrieben. Materialien, die diese Bedingung erfüllen, haben, wie sich gezeigt hat, die aus der Wunde abgesonderte Flüssigkeit gut aufgenommen. Hierbei ist es wichtig, daß das Material die abgesonderte Wundenflüssigkeit nicht nur absorbiert, sondern diese möglichst weit in den Absorptionskern 8 hinauf weiterleitet. Tatsächlich kann ein Material eine große Wasserabsorptionskraft ohne die gleichzeitige Fähigkeit haben, das Wasser auf eine bestimmte, beabsichtigte Höhe aufzusaugen. Diese letztgenannte Art von Absorptionsmaterial kann in der erfindungsgemäßen Schicht 10 nicht verwendet werden.

Wenn das Absorptionsmaterial keine zufriedenstellende Saughöhe hat, besteht die Gefahr einer starken Akkumulierung von Wundenflüssigkeit in dem die Wunde angrenzenden Teil des Absorptionskernes, was wiederum/zu einer Koagulierung von leicht Wundenflüssigkeit führt und deren weitere Absorptions verhindert. Es hat sich als vorteilhaft erweisen, den Absorptionskern 8 aus einem faserartigen Material, z.B. aus/Cellu- von losehalbstoff hergestelltem Flaum, zu arbeiten. Üblicher Flaum ("fluff"), der aus trocken-desintegriertem chemischem Cellulosehalbstoff, wie Sulfit- und Sulfathalbstoff, besteht, besitzt nicht die notwendige Saughöhe gemäß Klemm. Dagegen erfüllt ein aus einem solchen chemischen Halbstoff hergestellter Flaum die Forderung einer zufriedenstellenden Saughöhe, wenn der Halbstoff mit einem Vernetzungsmittel für Cellulose, wie z.B. Epichlorhydrin, vernetzt worden ist. Es wurde gefunden, daß die meisten Halbstoffe eine zusätzliche Epichlorhydrindmenge von 10 %, bezogen auf einen absolut trockenen Halbstoff, erfordern, um eine befriedigende Saughöhe zu erreichen. Die erfindungsgemäß geeigneten, ver-

netzten Cellulosehalbstoffe können z.B. nach dem Verfahren der US PS 3 700 549 hergestellt werden. Selbstverständlich können zur Vernetzung der Cellulose auch andere Vernetzungsmittel aus der Gruppe von Polyhalogeniden, Polyepoxiden und Epoxyhalogeniden verwendet werden.

Beispiele verschiedener, vernetzter Cellulosehalbstoffe und ihre Eigenschaften sind in der folgenden Tabelle 1 aufgeführt.

## Tabelle 1

| Halbstoff (Pulpe) | Test Nr. | Holländer Umdr. | Saughöhe mm/10 min |
|---|---|---|---|
| HWB Ausgangs-halbstoff | 354 | 500 | 36 |
| | 355 | 1000 | 25 |
| | 353 | 2000 | 7.5 |
| HWB 10% Epichlor-hydrin | 721 | 1000 | 51 |
| | 720 | 2000 | 31 |
| | 722 | 4000 | 12 |
| HWB 20% Epichlor-hydrin | 724 | 1000 | 88 |
| | 723 | 2000 | 50 |
| | 725 | 4000 | 20 |
| HBK Ausgangs-halbstoff | 364 | 1000 | 40 |
| | 362 | 2000 | 12 |
| | 363 | 4000 | 6 |
| HBK 10% Epichlor-hydrin | 679 | 1000 | 69 |
| | 680 | 2000 | 51 |
| | 681 | 4000 | 25 |
| HBK 20% Epichlor-hydrin | 682 | 1000 | 76 |
| | 683 | 2000 | 74 |
| | 684 | 4000 | 37 |

<u>Tabelle 1 Fortsetzung</u>

| Halbstoff | Test Nr. | Holländer Umdr. | Saughöhe mm/10 min |
|---|---|---|---|
| HK **Ausgangs-halbstoff** | 372 | 2000 | 28 |
| | 371 | 4000 | 8 |
| | 373 | 7000 | < 5 |
| HK 5% Epichlor-hydrin | 727 | 1000 | 85,5 |
| | 726 | 2000 | 63 |
| | 728 | 4000 | 40 |
| | 735 | 7000 | 17 |
| HK 10% Epichlor-hydrin | 730 | 1000 | 140 |
| | 729 | 2000 | 118 |
| | 731 | 4000 | 82 |
| | 736 | 7000 | 43 |
| MDC **Ausgangs-halbstoff** | 1045 | 500 | 19 |
| | 1044 | 1000 | 10 |
| | 1043 | 2000 | 5 |
| MDC 10% Epichlor-hydrin | 1030 | 500 | 53 |
| | 1029 | 1000 | 33,5 |
| | 1028 | 2000 | 16 |
| MDC 20% Epichlor-hydrin | 1032 | 1000 | 66 |
| | 1031 | 2000 | 28 |
| | 1033 | 4000 | 11 |

In der obigen Tabelle bedeuten:

HWB einen aus Birke hergestellten, gebleichten Sulfathalbstoff

HBK " " Kiefer* " " "

HK " " " " ungebleichten "

MDC " " Fichte " gebleichten "

Die Wasserabsorption gemäß Klemm (SCNA-P 13:64) gibt die Saughöhe in mm pro 10 Minuten für Wasser in einen vertikal aufgehängten Papierstreifen, dessen unterer Teil in das Wasser gehängt ist. Zur Herstellung des Papieres, das später in Streifen unterteilt wird, muß der Halbstoff * "pine"

gemahlen werden. Die Tabelle zeigt, daß die Saughöhe mit dem Mahlen variiert. Weiter ist ersichtlich, daß keiner der Ausgangshablstoffe eine erfindungsgemäß annehmbare Saughöhe gemäß Klemm, d.h. eine solche über 50 mm pro 10 Minuten, unabhängig vom Schlagen hat. Werden die Halbstoffe dagegen mit 10 % Epichlorhydrin oder mehr (wobei im Fall von HK 5 % Epichlorhydrin ausreichen) und durch Schlagen bei 500 oder 1000 Umdrehungen (in einer PFI-Mühle) behandelt, dann erreicht man eine Saughöhe über 50 mm pro 10 Minuten.

In einer bevorzugten Ausführungsform der vorliegenden Erfindung besteht der Absorptionskern 8 nur aus einem aus vernetztem Cellulosehalbstoff hergestellten Flaum mit der oben genannten hohen Wasserabsorptionsfähigkeit. In bestimmten Fällen kann es jedoch ausreichen, daß nur die der Wunde am nächsten liegende Flaumschicht aus vernetztem Cellulosehalbstoff besteht, während der restliche Absorptionskern aus Flaum besteht, der aus einem nicht-vernetzten Cellulosehalbstoff hergestellt ist und daher keine Saughöhe gemäß Klemm über 50 mm pro 10 Minuten zeigt, wie z.B. einige der in Tabelle I angegebenen Ausgangshalbstoffe. Die Dicke der Absorptionsschicht aus vernetztem Celluloseflaum sollte jedoch mindestens etwa ein Drittel der Dicke des gesamten Absorptionskernes ausmachen.

Weiter ist es erfindungsgemäß möglich, in die der Wunde benachbarten Flaumschicht ein hoch absorbierendes Material in Pulverform der oben beschriebenen Art, wie z.B. Dextran, einzumischen. Hierdurch kann ein Teil der guten Eigenschaften dieses Materials bei gleichzeitiger Vermeidung der Nachteile ausgenutzt werden. Z.B. können die Kosten erheblich gesenkt werden, da die in den Flaum eingemischte Menge an hoch absorbierendem Pulver bzw. Puder nur ein Bruchteil der/Menge ist, notwendigen wenn das Pulver direkt auf die Wunde aufgebracht wird. Die Neigung des Pulvers oder Puder zum Gelieren und zur Krustenbildung, wenn es bei direkter Aufbringung auf die Wunde nicht vor Sättigung mit Wundenflüssigkeit gewechselt wird, wird erfindungsgemäß auch vermieden, weil das Pulver zwischen den Fasern dispergiert ist und so eine fortgesetzte Absorption von Wundenflüssigkeit, auch nach Sättigung des Pulver mit dieser, möglich ist. Unter diesen Umständen wird die Absorption vollständig durch die Fasern aus vernetzter Cellulose übernommen, die zwischen den Pulverkörnern anwesend sind, wobei diese Fasern, wie oben erwähnt, eine hohe Absorptionsfähigkeit für Wundenflüssigkeit und gut für den Weitertransport über einen langen Weg in die Absorptionskörper hinauf sorgen können.

Weitere geeignete Absorptionsmaterialien sind z.B. Alginate. Neben den oben genannten Absorptionsmaterialien von organischem Ursprung kann der Absorptionskern 8 auch anorganische Absorptionsmaterialien enthalten, die die oben genannten Forderungen bezüglich Saughöhe erfüllen, wenn diese nicht toxisch sind oder keine allergischen Reaktionen bewirken.

Weiterhin kann man in den Absorptionskörper auch Substanzen mit bakterizider Wirkung, wie z.B. Chlorhexidin, einführen.

Die Dimensionen des Absorptionskörpers 5 und der verbindenden Folie 4 sowie der Klebestücke 1 und 2 sollten der Größe der zu behandelnden Wunde angepaßt sein, wobei unterschiedliche Größen vorkommen. Es wurde jedoch gefunden, daß drei Größen des Absorptionskörpers 5 besonders zweckmäßig sind, nämlich zwei Rechtecke mit den Dimensionen von etwa 8 x 5 cm bzw. etwa 10 x 8 cm und ein Viereck mit den Dimensionen 10 x 10 cm. Die Dicke des dicksten Teiles des Absorptionskörpers kann variieren und beträgt vorzugsweise zwischen etwa 1 und 4 cm.

Wie oben beschrieben, wird der erfindungsgemäße Verband in einer solchen Weise verwendet, daß die beiden Klebestücke 1 und 2 sowie die vorzugsweise durchsichtige Folie 4 an und über der Wunde angebracht und über längere Zeit dort gehalten werden. Der Absorptionskörper 5 sollte dagegen nach Bedarf gewechselt werden. Dies hat viele Vorteile; z.B. kann man die Wunde jederzeit durch einfache Lösen der Pflasterstücke 6 und 7 und Entfernung des Absorptionskörpers 5 untersuchen. Die Aufbringung des Verbandes und der Wechsel das Absorptionskörpers 5 können zuhause ohne Hilfe von medizinisch erfahrenem Personal erfolgen, da die Wunde selbst nie berührt zu werden braucht. Bei der Untersuchung der Wunde kann der Patient selbst feststellen, ob er die Hilfe eines Krankenhauses in Anspruch nehmen muß. Im Gegensatz dazu muß bei der Untersuchung von Wunden, auf die ein hoch absorbierender Puder direkt aufgebracht ist, zuerst der Verband, der das Puder auf der Wunde an Ort und Stelle hält, entfernt werden, worauf der Puder selbst entfernt werden muß, was oft Beschwerden macht und vorzugsweise durch medizinisches Personal durchgeführt werden sollte. Neben dem Vorteil des erfindungsgemäßen Verbandes, daß dieser vom Patienten selbst zuhause angelegt werden kann, wird auch in Krankenhäusern durch Verwendung des erfindungsgemäßen Verbandes die Wundbehandlung erleichtert, da der Verband leicht und praktisch zu handhaben ist. Außerdem werden, wie oben erwähnt, die Gesamtkosten für eine Wundheilung verringert.

<u>P a t e n t a n s p r ü c h e</u>

1.- Verband zur Aufbringung auf Wunden, insbesondere mit
starker Flüssigkeitssekretion, umfassend in Kombination:

a) zwei Klebestücken (1,2) mit halbkreisförmigen Aussparungen
zum Aufkleben auf die Haut oder gegebenenfalls teilweise
aufeinander, so daß die halbkreisförmigen Aussparungen die
Wunde einschließen,

b) eine vorzugsweise durchsichtige, perforierte Folie oder
ein Netz (4), das auf und gegebenenfalls auch außerhalb
der Klebestücke angeordnet ist, und an diesen und/oder der
Haut außerhalb der Klebestücke befestigt wird,

c) einem austauschbaren Absorptionskörper (5) von konvexer
Form in Bezug zur Wunde, bestehend aus einem Absorptionskern (8), der mindestens teilweise und in dem Gebiet um
die Wunde aus einem Material (10) mit einer Wasserabsorption von mindestens 50 mm pro 10 Minuten, gemessen gemäß
Klemm (SCAN-P 13:64), und einer flüssigkeitsdurchlässigen,
den Absorptionskern einschließenden Schicht (9), wobei der
Absorptionskörper auf der an die Wunde anschließende
Kunststoffolie oder das Kunststoffnetz angeordnet und mittels Klebestücken (6,7) in dieser Stellung befestigt ist.

2.- Verband nach Anspruch 1, dadurch gekennzeichnet, daß
der Absorptionskern aus einem faserartigen Material, insbesondere Celluloseflaum, besteht.

3.- Verband nach Anspruch 1-2, dadurch gekennzeichnet, daß
der Absorptionskern (8) vollständig aus einem Flaum besteht,
der aus einem vernetzten Cellulosehalbstoff mit der oben
genannten, hohen Wasserabsorptionsfähigkeit hergestellt ist.

4.- Verband nach Anspruch 1-2, dadurch gekennzeichnet, daß
der Absorptionskern (8) aus zwei Schichten von faserartigem
Material besteht und die an die Wunde angrenzende Schicht
aus einem Flaum besteht, der aus vernetztem Cellulosehalbstoff mit der oben genannten hohen Wasserabsorptionsfähigkeit hergestellt ist, während die nicht an die Wunde grenzende Schicht aus einem Flaum besteht, der aus nicht-vernetztem
Cellulosehalbstoff mit einer Wasserabsorption unter 50 mm pro 10 Minuten, gemessem nach Klemm, hergestellt ist.

5.- Verband nach Anspruch 3 und 4, dadurch gekennzeichnet, daß der an die Wunde angrenzende Flaum mit einem hoch absorbierenden Material in Pulver- bzw. Puderform gemischt ist.

## Fig. a

1    3    2

## Fig. b

4

## Fig. c

5

9    8

7    6

10   1    3    2    4

| | Europäisches Patentamt | EUROPÄISCHER RECHERCHENBERICHT | | Nummer der Anmeldung<br>EP 78 101 619. |

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | <u>DE - A - 2 045 941</u> (G. SORGATO)<br>* Seite 7, Zeilen 13 bis 20;<br>Fig. 1 *<br><br>-- | 1 |
| X | <u>US - A - 2 722 220</u> (M.A. MESTRAND)<br>* Spalte 2, Zeilen 22 bis 32;<br>Fig. 2 *<br><br>-- | 1 |
| X | <u>DE - A - 1 914 096</u> (K.H. SCHLICHT-<br>MANN)<br>* Seite 3, vorletzter Absatz bis<br>Seite 4, Zeile 5; Ansprüche 1<br>und 2; Fig. 1 *<br><br>-- | 1 |
| X | <u>DE - A - 2 012 362</u> (W. SEEFELD)<br>* Seiten 3 und 4; Fig. 1 *<br><br>-- | 1,2 |
| | <u>DE - C - 519 353</u> (E. SANDER)<br>* gesamtes Dokument *<br><br>-- | 1 |
| D | <u>US - A - 3 700 549</u> (J.L.-A. CROON<br>et al)<br>* gesamtes Dokument *<br><br>--<br>./.. | 2,3 |

**KLASSIFIKATION DER ANMELDUNG (Int.Cl.²)**

A 61 F 13/02
A 61 L 15/06

**RECHERCHIERTE SACHGEBIETE (Int. Cl.²)**

A 61 F 13/00
A 61 F 13/02
A 61 L 15/00
A 61 L 15/01
A 61 L 15/06

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort<br>Berlin | Abschlußdatum der Recherche<br>05-05-1979 | Prüfer<br>DROPMANN |

EPA form 1503.1  06.78

0002509

| Kategorie | EINSCHLÄGIGE DOKUMENTE | | KLASSIFIKATION DER ANMELDUNG (Int.Cl.²) |
|---|---|---|---|
| | Kennzeichnung des Dokuments mit Angabe, soweit erforderlich, der maßgeblichen Teile | betrifft Anspruch | |
| A | <u>DE - A - 2 448 664</u> (MINNESOTA MINING AND MANUFACTURING) <br> * Fig. 4 * | | |
| A | <u>DE - C - 513 235</u> (K. HARTMANN) <br> * gesamtes Dokument * | | RECHERCHIERTE SACHGEBIETE (Int. Cl.²) |

EPA Form 1503.2 06.78